# EUROPÄISCHE PATENTANMELDUNG

(11) **EP 0 715 846 A1**
(43) Veröffentlichungstag der Anmeldung: **12.06.1996**
(21) Anmeldenummer: 95118096.7
(22) Anmeldetag: 17.11.1995
(51) Int. Cl.: A61K 9/00, A61K 9/20

(54) **Pharmazeutische, oral anwendbare Zubereitung mit Aminosäure**

(30) Priorität: 10.12.1994 DE 4444052
(71) Anmelder: RHONE-POULENC RORER GMBH, 50792 Köln (DE)
(72) Erfinder: Jettka, Winfried, Dr., D-50765 Köln (DE); Gajdos, Benedikt, Dr., D-50767 Köln (DE); Dürr, Manfred, Dr., D-50129 Bergheim Glessen (DE)
(74) Vertreter: Döring, Wolfgang, Dr. Ing.

(57) **Zusammenfassung**

Es wird eine pharmazeutische, oral anwendbare Zubereitung beschrieben, wobei die feste Zubereitung mindestens einen antaciden Wirkstoff und/oder einen H₂-Antagonisten, mindestens ein Sprengmittel sowie mindestens einen pharmazeutisch üblichen Zuschlagstoff aufweist. Desweiteren enthält die Zubereitung mindestens einen, den Zerfall der Zubereitung im Mund oder in einer Flüssigkeit beschleunigenden Inhaltsstoff, wobei dieser Inhaltsstoff eine wasserlösliche Aminosäure, ein wasserlösliches Aminosäurederivat und/oder ein wasserlösliches Salz einer Aminosäure ist.

## Beschreibung

Die vorliegende Erfindung betrifft eine pharmazeutische, oral anwendbare Zubereitung mit den Merkmalen des Oberbegriffs des Patentanspruchs 1.

Feste, oral anwendbare pharmazeutische Zubereitungen, die mindestens einen antaciden Wirkstoff und/oder einen H₂-Antagonisten, ein Sprengmittel sowie mindestens einen pharmazeutisch üblichen Zuschlagsstoff aufweisen, sind seit langem bekannt und im Handel erhältlich. Abhängig von dem jeweiligen Wirkstoff bzw. dem Wirkstoffgemisch werden diese bekannten Antacide als Tablette, Dragee oder Pulver zur Vorbeugung und/oder zur Behandlung von Magenbeschwerden, insbesondere zur Behandlung bei Übelkeit, Magenkrämpfen, Sodbrennen, Völlegefühl, saures Aufstoßen, Erbrechen, Blähungen, Magengeschwüren und/oder bei Beschwerden nach Alkoholabusus bzw. Nikotinmißbrauch, angewendet. Hierfür ist es in der Regel erforderlich, daß der Patient die entsprechende Tablette oder das Dragee vor dem Verschlucken zerkaut, was vielfach zu einem Verkleben von Bestandteilen der Tablette bzw. des Dragee im Zahn- und/oder Gaumenbereich des Mundes führt. Diese verklebten Bestandteile lassen sich nur schwer entfernen und setzen darüber hinaus noch im Laufe der Zeit den Wirkstoff frei, was letztendlich dann einen äußerst unangenehmen Geschmack hervorruft.

Um die zuvor beschriebenen Probleme beim Einnehmen der bekannten pharmazeutischen Zubereitungen zu umgehen, sind speziell hergestellte Tabletten bekannt, bei denen der Zerfall im Mund oder in einer entsprechenden Flüssigkeit relativ schnell und zwangsläufig erfolgt, so daß hierbei ein Zerkauen der bekannten Tablette, die üblicherweise auch als Lyoc-Tablette bezeichnet wird, im Mund entfallen kann. Derartige spezielle und bekannte pharmazeutische Zubereitungen weisen jedoch den Nachteil auf, daß sie im Laufe ihrer Herstellung, während ihres Transportes zum Patienten oder bei ihrer Anwendung beim Patienten leicht und unerwünscht beschädigt werden können, so daß dementsprechend ihrer Verwendung ebenfalls eingeschränkt ist.

Der vorliegenden Erfindung liegt die Aufgabe zugrunde, eine oral anwendbare, feste Zubereitung der angegebenen Art zur Verfügung zu stellen, die einerseits eine besonders hohe Haltbarkeit und andererseits bei ihrer Anwendung sehr schnell zerfällt.

Diese Aufgabe wird erfindungsgemäß durch eine pharmazeutische Zubereitung mit den kennzeichnenden Merkmalen des Patentanspruchs 1 gelöst.

Die erfindungsgemäß pharmazeutische und oral anwendbare feste Zubereitung weist mindestens einen antaciden Wirkstoff und/oder ein H₂-Antagonisten, mindestens ein Sprengmittel sowie mindestens einen pharmazeutisch üblichen Zuschlagsstoff auf. Darüber hinaus enthält die erfindungsgemäße Zubereitung desweiteren mindestens einen, den Zerfall der Zubereitung im Mund oder in einer Flüssigkeit beschleunigenden Inhaltsstoff, wobei dieser Inhaltsstoff eine wasserlösliche Aminosäure, ein wasserlösliches Aminosäurederivat und/oder ein wasserlösliches Salz einer Aminosäure ist.

Überraschend konnte festgestellt werden, daß die erfindungsgemäße Zubereitung aufgrund des zuvor genannten mindestens einen Inhaltsstoffes (wasserlösliche Aminosäure, wasserlösliches Aminosäurederivat und/oder wasserlösliches Salz einer Aminosäure) eine sehr hohe Zerfallsrate, d.h. somit zeitlich gesehen einen sehr schnellen Zerfall, dann besitzt, wenn die erfindungsgemäße Zubereitung mit Speichel oder einer geeigneten Flüssigkeit, so insbesondere Wasser, in Kontakt kommt. Dies wiederum führt dazu, daß die erfindungsgemäße Zubereitung im Mund sehr schnell zerfällt, ohne daß es dabei erforderlich ist, die erfindungsgemäße Zubereitung durch Kauen zu zerkleinern. Folglich treten bei der erfindungsgemäßen Zubereitung nicht die Probleme auf, wie diese vorstehend bei den bekannten Zubereitungen, auch bei solchen Zubereitungen, die ein Sprengmittel enthalten, vorhanden sind, d.h. Bestandteile der erfindungsgemäßen Zubereitung setzen sich nicht in schwer zugänglichen Bereichen des Mundes ab und verursachen demnach auch kein unerwünschtes Verkleben auf der Zunge und/oder am Gaumen bzw. im Bereich der Zähne, so daß es dementsprechend bei der erfindungsgemäßen Zubereitung auch nicht dazu kommen kann, daß der jeweilige Patient bei Anwendung der erfindungsgemäßen Zubereitung einen bitteren Geschmack, hervorgerufen durch eine Freisetzung des Wirkstoffes, empfindet. Weiterhin wird durch den zuvor genannten und den Zerfall der Zubereitung im Mund oder in einer Flüssigkeit beschleunigenden Inhaltsstoff (wasserlösliche Aminosäure, wasserlösliches Aminosäurederivat und/oder wasserlösliches Salz einer Aminosäure) die Festigkeit der erfindungsgemäßen Zubereitung nicht verschlechtert, so daß es auch bei der erfindungsgemäßen Zubereitung nicht dazu kommen kann, daß die erfindungsgemäße Zubereitung bei ihrer Herstellung, dem Transport und ihrer Anwendung unerwünscht beschädigt wird, so daß dementsprechend die Reklamationsrate bei der erfindungsgemäßen Zubereitung besonders niedrig ist. Auch läßt sich die erfindungsgemäße Zubereitungen nach konventionellen Herstellungstechniken besonders preisgünstig produzieren, während dies bei der bekannten, zuvor genannten Lyoc-Tablette nicht der Fall ist.

Die zuvor angesprochene hohe Zerfallsrate der erfindungsgemäßen Zubereitung wird auf einen synergistischen Effekt des mindestens einen Sprengmittels mit dem Inhaltsstoff (wasserlösliche Aminosäure, wasserlösliches Aminosäurederivat und/oder wasserlösliches Salz einer Aminosäure) zurückgeführt.

Grundsätzlich kann die erfindungsgemäße Zubereitung jede wasserlösliche Aminosäure, jedes wasserlösliche Aminosäurederivat und/oder jedes wasserlösliches Salz einer Aminosäure enthalten, sofern sichergestellt ist, daß die zuvor genannten Inhaltsstoffe, die den Zerfall der erfindungsgemäßen Zubereitung im Mund oder in einer Flüssigkeit beschleunigen, untoxisch sind und keine Wechselwirkungen mit dem mindestens einen antaciden Wirkstoff bzw. H₂-Antagonisten eingehen. Besonders geeignet ist es, wenn die erfindungsgemäße Zubereitung als den Zerfall der Zubereitung beschleunigenden Inhaltsstoff, Glycin, ein Glycinderivat und/oder ein Glycinsalz enthält, wobei unter den Begriff Glycinderivat insbesondere Ester, vorzugsweise von C₁-C₄-Alkoholen, und/oder Amide von Glycin, vorzugsweise von C₁-C₁₀-Carbonsäuren, und unter den Begriff Glycinsalze vorzugsweise wasserlösliche Alkali- und/oder Erdalkalisalze sowie entsprechende Ammoniumsalze fallen. Diese zuvor beschriebenen Ausführungsformen der erfindungsgemäßen Zubereitung, die als Inhaltsstoff Glycin, ein Glycinderivat, ein Glycinsalz und/oder eine Mischung davon enthalten, sind toxikologisch völlig unbedenklich, wobei aufgrund des relativ geringen Preises der zuvor genannten und auf Glycin basierenden Inhaltsstoffe diese Ausführungsform der erfindungsgemäßen Zubereitung besonders preiswert herstellbar ist.

Eine andere Ausführungsform der erfindungsgemäßen pharmazeutischen Zubereitung weist zusätzlich zu den zuvor abgehandelten Inhaltsstoffen auf Glycin-Basis oder anstelle der zuvor genannten Inhaltsstoffe auf Glycin-Basis solche den Zerfall der Zubereitung beschleunigenden Inhaltsstoffe auf, die aus der Gruppe, bestehend aus Prolin, Hydroxyprolin und/oder Lysin sowie deren Salze und/oder Derivate, ausgewählt sind. Hierbei umfaßt der Begriff Salze und der Begriff Derivate die zuvor beim Glycin genannten Salze bzw. Derivate, wobei jedoch zusätzlich hierbei noch beim Prolin bzw. beim Hydroxyprolin die Möglichkeit besteht, den Pyrrolidin-Ring entsprechend zu substituieren, insbesondere zu halogenieren und/oder am Pyrrolidin-Ring eine zusätzliche NH₂-Gruppe, NO₂-Gruppe und/oder eine SO₃H-Gruppe vorzusehen. Ebenso kann an den nicht substituierten CH₂-Gruppen des Lysins einer oder mehrere der zuvor genannten Substituenten angeordnet werden.

Bezüglich der in der erfindungsgemäßen Zubereitung enthaltenen und vorstehend detailliert beschriebenen Inhaltsstoffen, die den Zerfall der erfindungsgemäßen Zubereitung im Mund oder einer Flüssigkeit beschleunigen, ist festzuhalten, daß die Inhaltsstoffe in einer solchen Konzentration in der erfindungsgemäßen Zubereitung enthalten sind, daß die Zubereitung im Mund bzw. in einer ausgewählten Flüssigkeit innerhalb von einer Sekunde bis sechzig Sekunden, vorzugsweise innerhalb von einer Sekunde bis dreißig Sekunden, zerfällt.

Abhängig von dem jeweils antaciden Wirkstoff bzw. H₂-Antagonisten sowie den üblichen Zuschlagsstoffen sowie der Aufmachung der festen erfindungsgemäßen Zubereitung variiert die Konzentration des Inhaltsstoffes bzw. des Gemisches der Inhaltsstoffes in der erfindungsgemäßen Zubereitung zwischen 1 Gew.% und 90 Gew.%, insbesondere zwischen 20 Gew.% und 70 Gew.% bezogen auf die anwendungsfertige Zubereitung.

Als Sprengmittel sind vorzugsweise in der erfindungsgemäßen Zubereitung Stärke, eine Stärkederivat, Cellulose, ein Cellulosederivat, Alginsäure, ein Alginsäurederivat, Casein, ein Caseinderivat und/oder Polyvinylpyrrolidon enthalten. Konkret handelt es sich bei der zuvor genannten Stärke insbesondere um Mais- oder Kartoffelstärke, bei dem zuvor genannten Stärkederivat insbesondere um modifizierte Stärke und/oder Natriumcarboxymethylstärke, auch in quervernetzter Form, bei dem zuvor genannten Cellulosederivat, insbesondere um Carboxymethylcellulose und/oder Calcium- und/oder Natriumcarboxymethylcellulose, auch in quervernetzter Form. Desweiteren eignen sich als Sprengmittel quervernetztes Casein, Natriumalginat sowie in Speichel und/oder Wasser unlösliches Polyvinylpyrrolidon (quervernetztes), wobei das letztgenannte Produkt auch unter der Bezeichnung Kollidon CL und Polyplasdone XL im Handel erhältlich ist.

Bezüglich der Konzentration des in der erfindungsgemäßen Zubereitung enthaltenen Sprengmittels ist festzuhalten, daß diese zwischen 1 Gew.% und 50 Gew.%, insbesondere zwischen 3 Gew.% und 20 Gew.%, bezogen auf die anwendungsfertige Zubereitung, variiert.

Eine besonders vorteilhafte Weiterbildung der zuvor beschriebenen Ausführungsform der erfindungsgemäßen Zubereitung sieht vor, daß hierbei die Zubereitung den mindestens einen antaciden Wirkstoff in Form von gleichmäßig in der festen Zubereitung verteilten Wirkstoffpartikeln aufweist. Insbesondere dann, wenn die antaciden Wirkstoffpartikel bzw. die Partikel des H₂-Antagonisten eine Größe zwischen 10 µm und 1.000 µm, vorzugsweise zwischen 50 µm und 400 µm, besitzen, weist eine derartige Weiterbildung der erfindungsgemäßen Zubereitung neben einer ausgezeichneten Haltbarkeit, einer besonders schnellen Zerfallsrate auch eine besondere hohe pharmazeutische Wirksamkeit auf. Dies hängt damit zusammen, daß nach Zerfall der erfindungsgemäßen Zubereitung die dann in den Magen gelangenden einzelnen Wirkstoffpartikel eine relativ große Oberfläche besitzen, so daß sie dementsprechend schnell die erwünschte therapeutische Wirkung hervorrufen können.

Bezüglich des in der erfindungsgemäßen Zubereitung enthaltenen antaciden Wirkstoffes bzw. des antacides Wirkstoffgemisches ist festzuhalten, daß es sich hierbei um die an sich bekannten antaciden Wirkstoffe handelt, vorzugsweise um Aluminiumhydroxid, Magnesiumhydroxid, Magnesiumtrisilicat, Magnesiumcarbonat, Magnesiumphosphat, Calciumcarbonat, Calciumphosphat, Natriumcitrat, Magnesiumoxid, Magaldrat, Hydrotalcit, Natriumhydrogencarbonat und/oder Wismutsubcarbonat.

Bezüglich der Konzentration der zuvor genannten antaciden Wirkstoffe in der erfindungsgemäßen Zubereitung ist festzuhalten, daß diese Konzentration des Wirkstoffes bzw. Wirkstoffgemisches zwischen 1 Gew.% und 70 Gew.%, insbesondere zwischen 15 Gew.% und 60 Gew.%, jeweils bezogen auf die anwendungsfertige Zubereitung, variiert.

Insbesondere dann, wenn die erfindungsgemäße Zubereitung als Wirkstoff einen hydrophilen antaciden Wirkstoff bzw. einen hydrophilen H₂-Antagonisten enthält, empfiehlt es sich, diesen hydrophilen Wirkstoff mit einer hydrophoben Beschichtungsschicht zu versehen und/oder den hydrophilen Wirkstoff in einer hydrophoben Matrix einzubetten. Diese Ausführungsform der erfindungsgemäßen Zubereitung weist nicht nur die bereits vorstehend genannten Vorteile (hohe Zerfallsrate, ausreichende Härte und Beständigkeit) auf, sondern zeichnet sich insbesondere auch noch dadurch aus, daß beim Zerfall der erfindungsgemäßen Zubereitung im Mund der Wirkstoff bzw. die konkreten Wirkstoffpartikel durch die hydrophobe Beschichtungsschicht bzw. die hydrophobe Matrix daran gehindert wird bzw. werden, sich zu größeren Agglomeraten zusammenzuballen, was der erwünschten Feinverteilung des Wirkstoffes entgegenstehen würde. Desweiteren wird durch eine derartige hydrophobe Beschichtungsschicht bzw. Matrix erreicht, daß ein möglicherweise vom Wirkstoff ausgehender bitterer oder unangenehmer Geschmack unterdrückt wird.

Die zuvor erwähnte Unterdrückung des bitteren und/oder unangenehmen Geschmackes des Wirkstoffes unter Beibehaltung einer besonders kurzen Zerfallszeit kann auch dadurch erreicht werden, daß die erfindungsgemäße Zubereitung einen Wirkstoff und/oder ein Wirkstoffgemisch aufweist, der bzw. das nicht mit einer hydrophoben Beschichtungsschicht versehen bzw. in einer entsprechenden Matrix eingebettet ist, sondern stattdessen größere, zuvor kompaktierte Wirkstoffteilchen oder auf eine entsprechende Teilchengröße granulierte Wirkstoffpartikel aufweist. Hierbei handelt es sich insbesondere um solche kompaktierten bzw. granulierten Wirkstoffteilchen, die ein Gemisch des Wirkstoffes mit einem Hilfsstoff, insbesondere einem Zucker und/oder Zuckeralkohol, darstellen. Durch ein derartiges Kompaktieren bzw. Granulieren, insbesondere durch ein Zusammenpressen, der Wirkstoffteilchen zu größeren Agglomeraten wird dann die zur Verfügung stehende Oberfläche im Vergleich zu konkreten, kleinen Wirkstoffpartikeln verkleinert, so daß dementsprechend der Auflösevorgang des eigentlichen Wirkstoffes, nicht jedoch der festen Zubereitung insgesamt, im Mund verzögert wird, was wiederum den unangenehmen oder bitteren Geschmack unterdrückt.

Grundsätzlich können zu der zuvor angesprochenen Beschichtung bzw. Einbettung des Wirkstoffes bzw. des Wirkstoffgemisches alle Beschichtungsmittel bzw. Einbettungsmittel ausgewählt werden, die einerseits die erwünschte Hydrophobierung des Wirkstoffes bzw. des Wirkstoffgemisches sicherstellen und andererseits toxisch unbedenklich sind. Hierfür kommen insbesondere Beschichtungsschichten bzw. Einbettungsmittel in Frage, die aus der Gruppe, bestehend aus Schellack, Stearinsäure, Gelatine, Zein, Gummi arabicum, Cellulosederivate, polymere Acrylsäurederivate und/oder polymere Vinylacetate ausgewählt sind. Konkret sind hier Methylcellulose, Ethylcellulose, Hydroxypropylcellulose, Hydroxypropylmethylcellulose, Celluloseacetat-Phthalat, Hydroxypropylmethylcellulosephthalat sowie polymere Acrylsäurederivate, insbesondere Copolymerisate aus Methacrylsäure und Estern von Methacrylsäuren, Acrylsäureethyl-Methacrylsäuremethylester-Copolymerisate, Methacrylsäure-Acrylsäuremethylester-Copolymerisate, Acryl- und Methacrylsäureester-Copolymerisate mit Trimethylammoniummethacrylat, Copolymerisate von Dimethylaminomethacrylsäure und neutralen Methacrylsäureestern, Vinylpyrrolidon-Vinylacetat-Copolymerisate, Polyvinylacetate, Polyvinylpyrrolidon, sowie Polyethylenglycol zu nennen.

Bezüglich der Mengen der zuvor genannten Beschichtungs- bzw. Einbettungsmittel ist festzuhalten, daß diese Menge der Beschichtungs- bzw. Einbettungsmittel abhängig von der Konzentration des jeweiligen hydrophilen Wirkstoffes bzw. des hydrophilen Wirkstoffgemisches zwischen 1 Gew.% und 10 Gew.%, bezogen auf die anwendungsfertige erfindungsgemäße Zubereitung, variiert.

Eine Weiterbildung der zuvor beschriebenen Ausführungsform der erfindungsgemäßen Zubereitung weist vorzugsweise anstelle des mindestens einen antaciden Wirkstoff mindestens einen H₂-Antagonisten, insbesondere Cimetidin, Famotidin, Ranitidin und/oder Nizatidine, auf. Hierbei variiert die Konzentration des zuvor genannten H₂-Antagonisten zwischen 1 Gew.% und 75 Gew.%, insbesondere zwischen 5 Gew.% und 50 Gew.%, jeweils bezogen auf die anwendungsfertige Zubereitung.

Insbesondere eine Kombination aus antacidem Wirkstoff bzw. antaciden Wirkstoffgemisch und den zuvor genannten H₂-Antagonisten besitzt dann hervorragende prophylaktische und/oder therapeutische Eigenschaften und kann insbesondere zur Behandlung von Ulcuserkrankungen des Magens und/oder des Zwölffingerdarmes sowie bei Refluxösophagitis eingesetzt werden.

Desweiteren sind in der erfindungsgemäßen Zubereitung pharmazeutisch übliche Zuschlagsstoffe enthalten. Hierunter fallen insbesondere Füllmittel, Bindemittel, Gleitmittel, Feuchthaltemittel, Absorptionsmittel, Antistatika, Farbstoffe, Konservierungsmittel und Geschmacksstoffe.

Insbesondere weist die erfindungsgemäße Zubereitung zwischen 0 Gew.% bis 60 Gew.% pharmazeutisch übliche Füll- und Bindemittel, jeweils zwischen 0 Gew.% und 10 Gew.% Polyvinylpyrrolidon, Propylenglycol, Polyethylenglycol, Zucker, Zuckeralkohole, Xanthan Gum und/oder Guar Gum sowie eine übliche Konzentration an Geschmacksstoffen, insbesondere an Süßstoffen und/oder Aromen, auf.

Wie bereits vorstehend erwähnt wurde, weist die erfindungsgemäße pharmazeutische Zubereitung eine feste Form auf und liegt vorzugsweise als Tablette oder Granulat vor. Selbstverständlich ist es jedoch auch möglich, die erfindungsgemäße Zubereitung als relativ grobkörniges Pulver aufzumachen.

Vorteilhafte Weiterbildungen der erfindungsgemäßen Zubereitung sind in den Unteransprüchen angegeben.

Die erfindungsgemäße Zubereitung wird anhand von Ausführungsbeispielen näher erläutert.

Nachfolgend werden bei den Ausführungsbeispielen die Begriffe Kompaktierung, Sprühtrocknung und Feuchtgranulierung verwendet.

Unter dem Begriff Kompaktierung wird ein Verfahren verstanden, bei dem der Wirkstoff bzw. das Wirkstoffgemisch mit einem in den Beispielen erwähnten konkreten Zuschlagsstoff bzw. ein Zuschlagsstoffgemisch, unter Umständen unter Zugabe von Wasser, mit einer geeigneten Vorrichtung, insbesondere einem Walzenkompaktor oder einer Tablettenpresse, kompaktiert wird. Anschließend wird das Kompaktat zerkleinert und ggf. getrocknet. Sollte das Kompaktat einen zu hohen Feinkornanteil, insbesondere unter 80 µm, aufweisen, so wird dieser Feinkornanteil durch Siebung entfernt werden.

Unter dem Begriff Sprühtrocknung wird ein Verfahren verstanden, bei dem der Wirkstoff bzw. das Wirkstoffgemisch in Wasser gelöst bzw. dispergiert wird. Hierbei werden die in den Ausführungsbeispielen konkret genannten Zuschlagsstoffe zugesetzt. Anschließend wird die Dispersion bzw. Lösung in einem Sprühturm im Warmluftstrom bei einer Produkttemperatur zwischen 30 °C und 120 °C getrocknet.

Unter dem Begriff Feuchtgranulierung wird ein Verfahren verstanden, bei dem der Wirkstoff bzw. das Wirkstoffgemisch in einer geeigneten Vorrichtung, insbesondere einem Mischer bzw. Wirbelschichtgranulator, unter Zugabe von in den Beispielen erwähnten Zuschlagsstoffen granuliert. Abhängig von dem jeweils ausgewählten Wirkstoff und den Zuschlagsstoffen kann das Granulat mit einem geeigneten Polymeren beschichtet werden.

Werden die der Sprühtrocknung, der Feuchtgranulierung und der Kompaktierung unterworfenen Produkte mit einem geeigneten Polymer, bei dem es sich bei den nachfolgenden Beispielen um das Produkt Eudragit E 12,5 handelt, beschichtet, so ist dies bei den nachfolgend wiedergegebenen Ausführungsbeispielen durch Angabe des zuvor genannten Produktes gekennzeichnet.

Zu den nachfolgend in den Ausführungsbeispielen mit ihren Handelsnamen bezeichneten Bestandteilen, Kollidon CL (BASF), Aerosil 200 (Degussa), Acesulfam K (Hoechst) und Eudragit E12,5 (Röhm) sind jeweils in Klammern hinter dem einzelnen Bestandteil der Hersteller angegeben.

### Ausführungsbeispiel 1

### Herstellung einer Aluminiumhydroxid/Magnesiumhydroxid-haltigen Tablette.

Eine Mischung, die
- - 5,714 kg: wasserhaltiges Aluminiumoxid*,
- - 5,714 kg: Magnesiumhydroxid Pulver, wasserfrei,
- - 2,071 kg: Sorbitol Lösung 70 Gew.% (nicht krist.) und
- - q.s.: Wasser
enthielt, wurde zunächst durch intensives Vermischen hergestellt. Nachdem diese Mischung kompaktiert worden war, wurde das Kompaktat zerkleinert und getrocknet. Das hierbei entstehende Zwischenprodukt wurde mit den nachfolgend aufgeführten Bestandteilen
- - 5,357 kg: Glycin,
- - 1,228 kg: Kollidon CL (quervernetztes Povidon),
- - 95 g: Aerosil 200,
- - 16 g: Acesulfam K,
- - 61 g: Aromastoffe,
- - 261 g: Talkum und
- - 12 g: Magnesiumstearat
vermischt und die dabei entstehende homogene Mischung wurde zu Tabletten mit einem Gewicht von 1.400 mg verpreßt. Der Preßdruck betrug 30 kN bei einem Tabletten-Durchmesser von 16 mm.
q.s. bedeutet die Menge Wasser (ca. 1.000 ml), die notwendig ist, um einen für die Kompaktierung geeigneten Wassergehalt der Pulvermischung einzustellen (ca. 10 - 15 Gew.%).
* entspricht 2,857 kg Al₂O₃ (wasserfrei)

### Ausführungsbeispiel 2

### Herstellung einer Aluminiumhydroxid/Magnesiumhydroxid-haltigen Tablette.

Eine Mischung, die
- - 1.250 g: Aluminiumhydroxidgel*,
- - 1.000 g: Magnesiumhydroxid-Paste,
- - 543 g: Sorbitol 70 %ig, nicht kristallisiert,
- - 13,8 g: Povidon und
- - 1,8 g: Saccharin-Natrium
enthielt, wurde einer Sprühtrocknung bei einer Temperatur von 108 °C unterworfen. Das hierbei entstehende Sprühtrocknungsprodukt wurde mit
- - 36 g: Eudragit E 12,5
überzogen und anschließend mit den nachfolgend genannten Bestandteilen
- - 1200 g: Glycin,
- - 240 g: Kollidon CL (quervernetztes Povidon),
- - 10 g: Aerosil 200,
- - 6 g: Aromastoffe,
- - 50 g: Talkum und
- - 24 g: Magnesiumstearat
gründlich vermischt. Die hierbei entstehende homogene Mischung wurde zu Tabletten mit einem Gewicht von 1.380 mg und einem Durchmesser von 16 mm bei einem Preßdruck von 35 kN verpreßt .
* entspricht 200 g Al₂O₃ (wasserfrei)

### Ausführungsbeispiel 3

### Herstellung einer Aluminiumhydroxid/Magnesiumhydroxid-haltigen Tablette.

Eine Mischung, die
- - 800 g: wasserhaltiges Aluminiumoxid *,
- - 800 g: Magnesiumhydroxid-Pulver, wasserfrei,
- - 290 g: Saccharose,
- - 300 g: Mannitol,
- - 98 g: Sorbitol, 70 %ig, nicht kristallisierend und
- - 9 g: Saccharin-Natrium
enthielt, wurde durch Feuchtgranulierung hergestellt. Anschließend wurde das Granulat getrocknet und mit
- - 50 g: Eudragit E 12,5
überzogen. Zu dem überzogenen Granulat wurden die nachfolgenden Bestandteile
- - 1.200 g: Glycin,
- - 240 g: Kollidon CL,
- - 10 g: Aerosil 200,
- - 6 g: Aromastoffe,
- - 56 g: Talkum und
- - 14 g: Magnesiumstearat
zugemischt. Die homogene Mischung wurde zu Tabletten von einem Gewicht von 1936 mg und einem Durchmesser von 17,5 mm bei einem Preßdruck von 40 kN verpreßt.
* entspricht 400 g Al₂O₃ (wasserfrei)

### Ausführungsbeispiel 4

### Herstellung einer Magaldrat-haltigen Tablette.

Eine Mischung, die
- - 800 g: Magaldrat und
- - 143 g: Sorbitol Lösung 70 Gew.% (nicht kristallisierend)
enthielt, wurde hergestellt. Anschließend wurde diese Mischung walzenkompaktiert. Das kompaktierte Gemisch wurde zerkleinert, getrocknet und mit den nachfolgend aufgeführten Bestandteilen
- - 375 g: Glycin,
- - 100 g: Kollidon CL,
- - 6,7 g: Aerosil 200,
- - 1,1 g: Acesulfam K,
- - 4,3 g: Aromastoffe,
- - 18,3 g: Talkum und
- - 8,6 g: Magnesiumstearat
vermischt. Die homogene Mischung wurde zu Tabletten mit einem Gewicht von 1.415 mg und einem Durchmesser von 16 mm bei einem Preßdruck von 30 kN verpreßt.

### Ausführungsbeispiel 5

### Herstellung einer Hydrotalcit-haltigen Tablette.

Eine Mischung, die
- - 1000 g: Hydrotalcit und
- - 140 g: Xylitol
enthielt, wurde hergestellt. Anschließend wurde diese Mischung walzenkompaktiert. Das kompaktierte Produkt wurde zerkleinert und getrocknet. Zu diesem getrockneten Produkt wurden die nachfolgenden Bestandteile
- - 400 g: Glycin,
- - 130 g: Kollidon CL,
- - 8,3 g: Aerosil 200,
- - 1,5 g: Acesulfam K,
- - 5,6 g: Aromastoffe,
- - 20 g: Talkum und
- - 9,3 g: Magnesiumstearat
zugemischt. Nach Homogenisierung wurde die Mischung zu Tabletten mit einem Gewicht von 1715 mg und einem Durchmesser von 17,5 mm bei einem Preßdruck von 35 kN verpreßt.

### Ausführungsbeispiel 6

### Herstellung einer Cimetidin-haltigen Tablette.

Eine Mischung, die
- - 400 g: Cimetidin,
- - 144 g: Sorbitollösung 70 Gew.% (nicht kristallisierend) und
- - 9 g: Saccharin-Natrium
enthielt, wurde hergestellt. Das walzenkompaktierte Produkt wurde granuliert und getrocknet. Anschließend wurde das getrocknete Produkt mit
- - 20 g: Eudragit E 12,5
beschichtet. Das beschichtete Produkt wurde hiernach mit den nachfolgenden Bestandteilen
- - 400 g: Glycin,
- - 80 g: Kollidon CL,
- - 6 g: Aerosil 200,
- - 4 g: Aromastoffe,
- - 20 g: Talkum und
- - 8 g: Magnesiumstearat
vermischt. Die homogenisierte Mischung wurde anschließend zu Tabletten mit einem Gewicht von 525 mg und einem Durchmesser von 12 mm bei einem Preßdruck von 10 kN verpreßt.

Nach den vorstehend in den Ausführungsbeispielen 1 bis 6 angegebenen Herstellungsvorschriften wurden jeweils weitere Tabletten I bis VI hergestellt, wobei diese weiteren Tabletten I bis VI jeweils kein Glycin aufwiesen. Vielmehr war die Menge an Glycin durch eine entsprechende Menge an Sorbitol ersetzt.

Von den vorstehend genannten und nach den Ausführungsbeispielen 1 bis 6 hergestellten sowie von den Vergleichstabletten I bis VI, die kein Glycin aufwiesen, wurden die Zerfallszeiten bestimmt. Hierfür wurde eine modifizierte Apparatur nach DAB 10 verwendet.

Die modifizierte Apparatur bestand aus einem Gestell mit Siebboden, das sechs zylindrische Prüfröhrchen aus Glas enthielt. Die kopfseitig offenen Röhrchen waren fußseitig lediglich durch ein Netz aus rostfreiem Stahldraht verschlossen, so daß die Flüssigkeit frei in das Röhrchen eindringen konnte. Das Gestell wurde durch einen Motor regelmäßig vertikal auf- und abwärts bewegt, wobei die Geschwindigkeit so eingestellt wurde, daß das Gestell 28 bis 32 mal pro Minute um jeweils 50 bis 60 mm auf- und abwärts bewegt wurde. Das Gestell wurde hierbei in einem 1-Liter-Becherglas angeordnet, wobei das Becherglas mit 350 ml gereinigtem Wasser gefüllt war. Die Positionierung des Gestells wurde so vorgenommen, daß die Röhrchen in der obersten Endstellung der Aufwärtsbewegung die Wasserschicht ganz verlassen hatten und in der untersten Endstellung der Abwärtsbewegung so weit in das Wasser eintauchten, daß alle, in den Röhrchen angeordneten Tabletten vollständig mit Wasser genetzt waren.

In jedem Röhrchen wurde jeweils eine Tablette angeordnet.

Die Temperatur des Wassers während der Messung wurde auf einen Wert zwischen 36 °C und 38 °C eingestellt.

Das Gestell wurde dann so lange auf- und abwärts bewegt, bis alle Prüflinge zerfallen waren. Die hierfür erforderliche Zerfallszeit wurde bestimmt, wobei in der nachfolgend wiedergegebenen Tabelle 1 diese Werte als Mittelwerte von jeweils dreißig Parallelbestimmungen angegeben sind.

**Tabelle 1**

| Zerfallszeiten der nach den Ausführungsbeispielen 1 bis 6 hergestellten Tabletten sowie der Tabletten I bis VI (ohne Zusatz von Glycin) | |
|---|---|
| Tablette gemäß Beispiel | Zerfallszeit in Sekunden |
| 1 | 10 |
| 2 | 18 |
| 3 | 20 |
| 4 | 15 |
| 5 | 15 |
| 6 | 12 |

| Vergleichstabletten | |
|---|---|
| I | ≧ 300 |
| II | ≧ 300 |
| III | ≧ 300 |
| IV | ≧ 300 |
| V | ≧ 300 |
| VI | ≧ 300 |

Es wurde ein Geschmacks- und Akzeptanzversuch mit dreißig Personen durchgeführt, die über mehrere Tage eine handelsübliche Tablette gemäß III oder ein äußerlich identisches tablettenförmiges Mittel (gemäß Ausführungsbeispiel 3) eingenommen haben, das sich von dem bekannten Mittel die zusätzlich in dem Ausführungsbeispiel 3 angegebene Glycin-Menge enthielt.

Beide Tabletten konnten von den Personen in ihrem Äußeren nicht unterschieden werden. Alle Personen erhielten sowohl die handelsübliche Tablette als auch das vorstehend genannte tablettenförmige Mittel.

Während und nach Abschluß der Testphase wurde von allen 30 Personen mitgeteilt, wie angenehm und unproblematisch die Anwendung der mit Glycin versetzten Tablette (gemäß Ausführungsbeispiel 3) gewesen war.

Insbesondere bestätigten die Personen übereinstimmend, daß im Vergleich zum herkömmlichen Antacidum die mit Glycin versetzte Tablette im Mund während des Einnehmens und Kauens als aüßerst positiv und angenehm empfunden wurde. Das typische, bei der bekannten Tablette immer auftretende Verkleben von Tablettenbestandteilen an Gaumen, Zähnen und Zahnfleisch konnte bei der mit Glycin versetzten Tablette nicht festgestellt werden. Auch löste sich diese Tablette wesentlich schneller auf, was als sehr komfortabel empfunden wurde.

## Patentansprüche

1. Pharmazeutische, oral anwendbare Zubereitung, wobei die feste Zubereitung mindestens einen antaciden Wirkstoff und/oder einen H₂-Antagonisten, mindestens ein Sprengmittel sowie mindestens einen pharmazeutisch üblichen Zuschlagstoff aufweist, dadurch gekennzeichnet, daß die Zubereitung desweiteren mindestens einen, den Zerfall der Zubereitung im Mund oder in einer Flüssigkeit beschleunigenden Inhaltsstoff enthält, wobei dieser Inhaltsstoff eine wasserlösliche Aminosäure, ein wasserlösliches Aminosäurederivat und/oder ein wasserlösliches Salz einer Aminosäure ist.

2. Pharmazeutische Zubereitung nach Anspruch 1, dadurch gekennzeichnet, daß der Inhaltsstoff Glycin, ein Glycinderivat und/oder ein Glycinsalz ist.

3. Pharmazeutische Zubereitung nach Anspruch 1 oder 2, dadurch gekennzeichnet, daß der Inhaltsstoff Prolin, Hydroxyprolin und/oder Lysin, ein Salz und/oder ein Derivat davon ist.

4. Pharmazeutische Zubereitung nach einem der vorangehenden Ansprüche, dadurch gekennzeichnet, daß die Zubereitung den Inhaltsstoff in einer solchen Konzentration aufweist, daß die Zubereitung im Mund oder in einer Flüssigkeit innerhalb von einer Sekunde bis sechzig Sekunden, insbesondere innerhalb von einer Sekunde bis dreißig Sekunden, zerfällt.

5. Pharmazeutische Zubereitung nach Anspruch 4, dadurch gekennzeichnet, daß die Zubereitung den Inhaltsstoff in einer Konzentration zwischen 1 Gew.% und 90 Gew.%, insbesondere in einer Konzentration zwischen 20 Gew.% und 70 Gew.%, bezogen auf die anwendungsfertige Zubereitung, aufweist.

6. Pharmazeutische Zubereitung nach einem der vorangehenden Ansprüche, dadurch gekennzeichnet, daß das Sprengmittel Stärke, ein Stärkederivat, Cellulose, ein Cellulosederivat, Alginsäure, ein Alginsäurederivat, Casein, ein Caseinderivat und/oder ein unlösliches Polyvinylpyrrolidon ist.

7. Pharmazeutische Zubereitung nach einem der vorangehenden Ansprüche, dadurch gekennzeichnet, daß die Zubereitung das Sprengmittel in einer Konzentration zwischen 1 Gew.% und 50 Gew.%, insbesondere in einer Konzentration zwischen 3 Gew.% und 20 Gew.%, bezogen auf die anwendungsfertige Zubereitung, aufweist.

8. Pharmazeutische Zubereitung nach einem der vorangehenden Ansprüche, dadurch gekennzeichnet, daß die Zubereitung den mindestens einen antaciden Wirkstoff in Form von gleichmäßig in der festen Zubereitung verteilten Wirkstoffpartikeln aufweist.

9. Pharmazeutische Zubereitung nach Anspruch 8, dadurch gekennzeichnet, daß die Wirkstoffpartikel eine Größe zwischen 10 µm und 1.000 µm, insbesondere zwischen 50 µm und 400 µm, besitzen.

10. Pharmazeutische Zubereitung nach einem der vorangehenden Ansprüche, dadurch gekennzeichnet, daß der mindestens eine antacide Wirkstoff Aluminiumhydroxid, Magnesiumhydroxid, Magnesiumtrisilicat, Magnesiumcarbonat, Magnesiumphosphat, Calciumcarbonat, Calciumphosphat, Natriumcitrat, Magnesiumoxid, Magaldrat, Hydrotalcit, Natriumhydrogencarbonat und/oder Wismutsubcarbonat ist.

11. Pharmazeutische Zubereitung nach einem der vorangehenden Ansprüche, dadurch gekennzeichnet, daß die Zubereitung den mindestens einen antaciden Wirkstoff in einer Konzentration zwischen 1 Gew.% und 70 Gew.%, insbesondere in einer Konzentration zwischen 15 Gew.% und 60 Gew.%, jeweils bezogen auf die anwendungsfertige Zubereitung, aufweist.

12. Pharmazeutische Zubereitung nach einem der vorangehenden Ansprüche, dadurch gekennzeichnet, daß die Zubereitung mindestens einen hydrophilen antaciden Wirkstoff enthält, der mit einer hydrophoben Beschichtungsschicht versehen ist.

13. Pharmazeutische Zubereitung nach Anspruch 12, dadurch gekennzeichnet, daß die hydrophobe Beschichtungsschicht Schellack, Stearinsäure, Gelatine, Zein, Gummi arabicum, Cellulosederivate, polymere Acrylsäurederivate und/oder polymere Vinylacetate umfaßt.

14. Pharmazeutische Zubereitung nach einem der vorangehenden Ansprüche, dadurch gekennzeichnet, daß die Zubereitung als Wirkstoff mindestens einen H₂-Antagonisten, insbesondere Cimitidin, Ranatidin und/oder Famotidin, enthält.

15. Pharmazeutische Zubereitung nach Anspruch 14, dadurch gekennzeichnet, daß die Zubereitung den mindestens einen H₂-Antagonisten in einer Konzentration zwischen 1 Gew.% und 75 Gew.%, insbesondere in einer Konzentration zwischen 5 Gew.% und 50 Gew.%, bezogen auf die anwendungsfertige Zubereitung, enthält.

16. Pharmazeutische Zubereitung nach einem der vorangehenden Ansprüche, dadurch gekennzeichnet, daß die feste Zubereitung zwischen 0 Gew.% bis 60 Gew.% Füll- und Bindemittel, jeweils zwischen 0 Gew.% und 10 Gew.% Polyvinylpyrrolidon, Propylenglycol, Polyethylenglycol, Zucker, Zuckeralkohole, Xanthan Gum und/oder Guar Gum sowie übliche Süßstoffe und/oder Aromen aufweist.

17. Pharmazeutische Zubereitung nach einem der vorangehenden Ansprüche, dadurch gekennzeichnet, daß der Wirkstoff aus unter Druck kompaktierten Wirkstoffteilchen oder aus einem unter Druck kompaktierten Teilchengemisch aus Wirkstoff und mindestens einem Hilfsstoff, besteht.

18. Pharmazeutische Zubereitung nach einem der vorangehenden Ansprüche, dadurch gekennzeichnet, daß die Zubereitung in Form einer Tablette oder eines Granulates vorliegt.
